(19) Europäisches Patentamt
European Patent Office
Office européen des brevets

(11) Veröffentlichungsnummer: **0 269 929**
**A1**

# (12) EUROPÄISCHE PATENTANMELDUNG

(21) Anmeldenummer: **87116766.4**

(22) Anmeldetag: **13.11.87**

(51) Int. Cl.⁴: **C07D 271/06**, C07D 413/12, A01N 43/82

(30) Priorität: **25.11.86 DE 3640153**

(43) Veröffentlichungstag der Anmeldung: **08.06.88 Patentblatt 88/23**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Heinemann, Ulrich, Dr.**
**Feldstrasse 18**
**D-5653 Leichlingen(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58a**
**D-5090 Leverkusen 3(DE)**

## (54) Chlor-1,2,4-oxadiazole.

(57) 3-Chlor-1,2,4-oxadiazole der allgemeinen Formel (I)

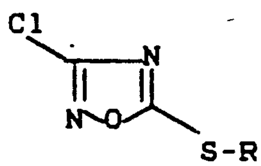

(I)

in welcher
R für gegebenenfalls substituiertes Aryl oder für gegebenenfalls substituiertes Heteroaryl steht,
und ihre Verwendung als Schädlingsbekämpfungsmittel.

Die neuen 3-Chlor-1,2,4-oxadiazole der allgemeinen Formel (I) können z.B. hergestellt werden, indem man 3,5-Dichlor-1,2,4-oxadiazol mit geeigneten Thiolen gegebenenfalls in Gegenwart eines Verdünnungsmittels und gegebenenfalls in Gegenwart eines Säurebindemittels umsetzt.

EP 0 269 929 A1

# Chlor-1,2,4-oxadiazole

Die Erfindung betrifft neue 3-Chlor-1,2,4-oxadiazole, ein Verfahren zu ihrer Herstellung sowie ihre Verwendung als Schädlingsbekämpfungsmittel.

Es ist bereits bekannt, daß bestimmte 3-Chlor-1,2,4-oxadiazole, wie beispielsweise der N-(3-Chlor-1,2,4-oxa-diazol-5-yl)-N'-(4-chlorphenyl)-harnstoff fungizide Eigenschaften besitzen (vgl. DE-OS 34 45 205).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue 3-Chlor-1,2,4-oxadiazole der allgemeinen Formel (I),

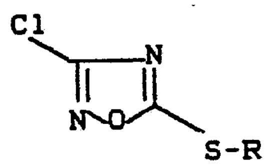

(I)

in welcher
R für gegebenenfalls substituiertes Aryl oder für gegebenenfalls substituiertes Heteroaryl steht,
gefunden.

Weiterhin wurde gefunden, daß man die neuen 3-Chlor-1,2,4-oxadiazole der allgemeinen Formel (I),

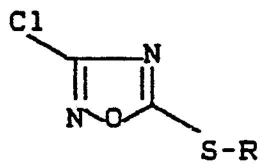

(I)

in welcher
R für gegebenenfalls substituiertes Aryl oder für gegebenenfalls substituiertes Heteroaryl steht,
erhält, wenn man 3,5-Dichlor-1,2,4-oxadiazol der Formel (II)

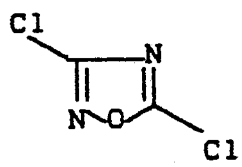

(II)

mit Thiolen der allgemeinen Formel (III),
HS-R (III)
in welcher
R die oben angegebene Bedeutung hat,
gegebenenfalls in Gegenwart eines Verdünnungsmittels sowie gegebenenfalls in Gegenwart eines Säurebindemittels umsetzt.

Schließlich wurde gefunden, daß die neuen 3-Chlor-1,2,4-oxadiazole der Formel (I) eine Wirkung gegen Schädlinge besitzen.

Überraschenderweise zeigen die erfindungsgemäßen 3-Chlor-1,2,4-oxadiazole der Formel (I) u.a. eine erheblich bessere fungizide Wirksamkeit als die aus dem Stand der Technik bekannten 3-Chlor-1,2,4-oxadiozole, wie beispielsweise der N-(3-Chlor-1,2,4-oxadiazol-5-yl)-N'-(4-chlorphenyl)-harnstoff, welches chemisch und wirkungsmäßig naheliegende Verbindungen sind.

Die erfindungsgemäßen 3-Chlor-1,2,4-oxadiazole sind durch die Formel (I) allgemein definiert. Bevorzugt sind Verbindungen der Formel (I), bei welchen
R für gegebenenfalls einfach bis mehrfach, gleich oder verschieden substituiertes Aryl mit 6 bis 10 Kohlenstoffatomen oder für gegebenenfalls einfach bis mehrfach, gleich oder verschieden substituiertes und/oder benzannelliertes 5-bis 7-gliedriges Heteroaryl mit 1 bis 4 gegebenenfalls gleichen oder verschiedenen Heteroatomen, insbesondere Stickstoff, Sauerstoff und/oder Schwefel steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Hydroxycarbonyl, Aminocarbonyl,

geradkettiges oder verzweigtes Alkoxycarbonyl mit 1 bis 5 Kohlenstoffatomen, Aryloxy oder Arylthio mit jeweils 6 bis 10 Kohlenstoffatomen, jeweils geradkettiges oder verzweigtes Dialkylamino oder Alkylcarbonylamino mit jeweils 1 bis 4 Kohlenstoffatomen in den einzelnen Alkylteilen oder ein gegebenenfalls einfach oder mehrfach, gleich oder verschieden durch niederes Alkyl und/oder Halogen substituierter 5-Ring-oder 6-Ring-Heterocyclus mit 1 bis 4 gegebenenfalls gleichen oder verschiedenen Heteroatomen, insbesondere Stickstoff, Sauerstoff und/oder Schwefel.

Besonders bevorzugt sind Verbindungen der Formel (I), bei welchen

R für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s-oder t-Butyl, n-oder i-Pentyl, Methoxy, Ethoxy, n-oder i-Propoxy, Methylthio, Ethylthio, n-oder i-Propylthio, Trifluormethyl, Trichlormethyl, Tribrommethyl, Trifluormethoxy, Trifluormethylthio, Hydroxycarbonyl, Aminocarbonyl, Methoxycarbonyl, Ethoxycarbonyl, Phenoxy, Phenylthio, Dimethylamino, Diethylamino, Methylcarbonylamino, Ethylcarbonylamino oder ein gegebenenfalls ein-oder zweifach, gleich oder verschieden durch Chlor, Brom, Methyl und/oder Ethyl substituierter 5-Ring-oder 6-Ring-Heterocyclus mit 1 bis 4 gegebenenfalls gleichen oder verschiedenen Heteroatomen, insbesondere Stickstoff, Sauerstoff oder Schwefel; außerdem für jeweils gegebenenfalls ein-bis dreifach, gleich oder verschieden substituiertes α -Naphthyl oder β -Naphthyl steht, wobei als Substituenten infrage kommen: Fluor, Chlor, Brom, Nitro, Methyl, Ethyl, n-oder i-Propyl, n-, i-, s-oder t-Butyl, Methoxy, Ethoxy, Methylthio oder Trifluormethyl;

außerdem für jeweils gegebenenfalls ein-bis dreifach, gleich oder verschieden substituiertes 2-Pyridyl, 4-Pyridyl, 2-Pyrimidyl, 4-Pyrimidyl, s-Triazinyl, 2-Chinolyl oder 1-Isochinolyl steht, wobei also Substituenten jeweils infrage kommen: Fluor, Chlor, Brom, Cyano, Nitro, Methyl, Ethyl, n-oder i-Propyl, n-, i-, s-oder t-Butyl, n-oder i-Pentyl, Methoxy, Ethoxy, Methylthio, Trifluormethyl, Trichlormethyl, Tribrommethyl, Trifluormethoxy, Trifluormethylthio, Hydroxycarbonyl, Amino-carbonyl, Methoxycarbonyl, Ethoxycarbonyl, Dimethylamino, Methylcarbonylamino oder Ethylcarbonylamino.

Im einzelnen seien außer den bei den Herstellungsbeispielen genannten Verbindungen die folgenden 3-Chlor-1,2,4-oxadiazole der allgemeinen Formel (I) genannt:

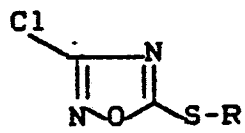 (I)

| R | R |
|---|---|
| 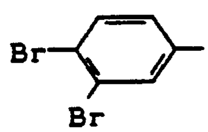 | 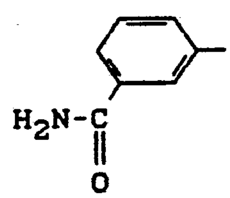 |
| 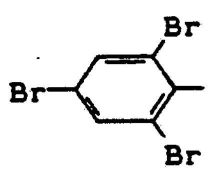 | 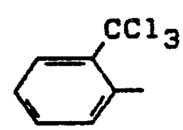 |
| 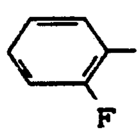 | 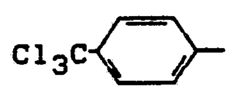 |
| 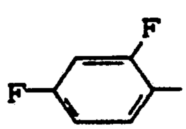 | 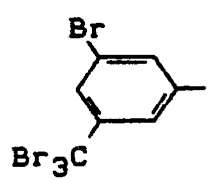 |

| R | R |
|---|---|
| 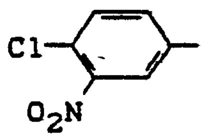 | 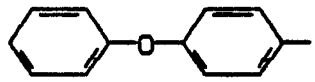 |
| 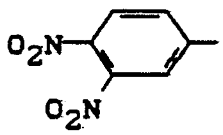 | 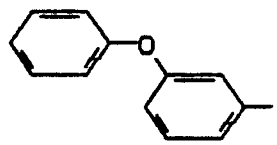 |
| 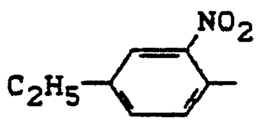 | 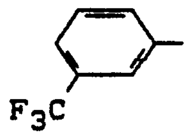 |
| 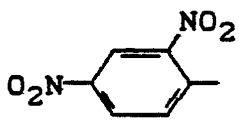 | 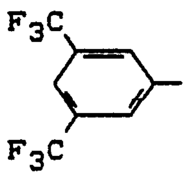 |
| 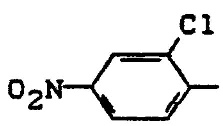 | 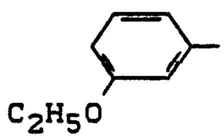 |
| 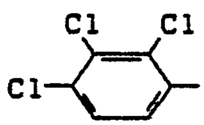 | 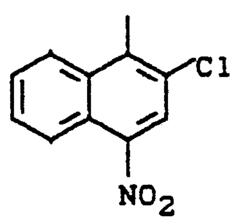 |
| 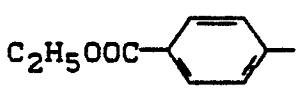 | 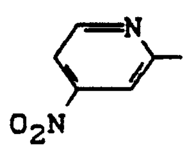 |
| 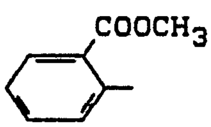 | |

| R | R |
|---|---|
| 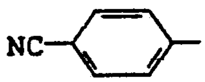 | 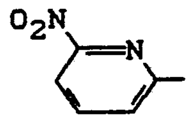 |
| 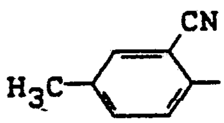 | 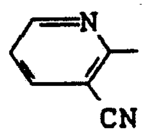 |
| 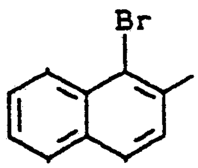 | 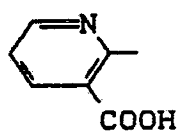 |
| 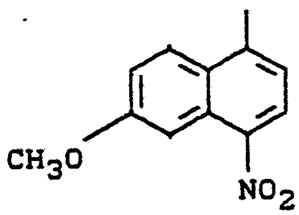 | 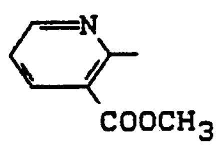 |
| 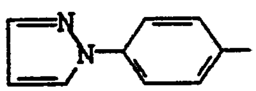 | 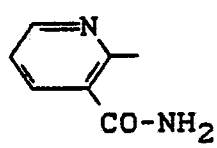 |
| 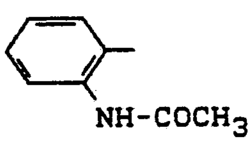 | 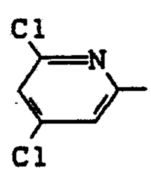 |
| 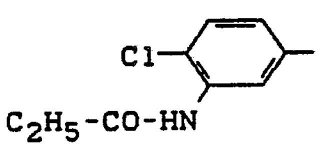 | 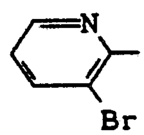 |

| R | R |
|---|---|
| 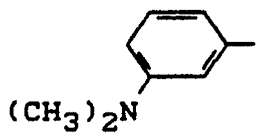 | 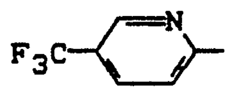 |
| 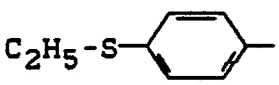 | 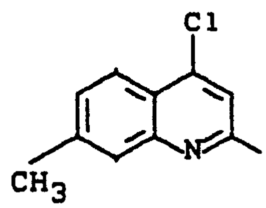 |
| 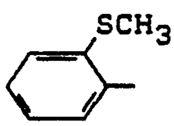 | 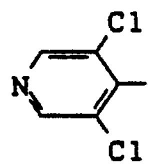 |
| 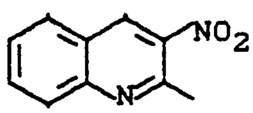 | 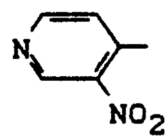 |
| 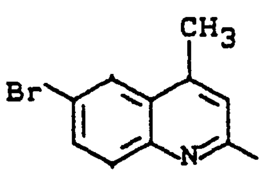 | |
| 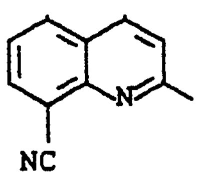 | |
| 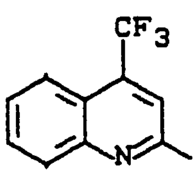 | |

| R | R |
|---|---|
| 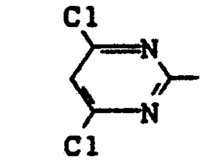 | 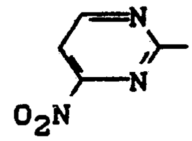 |
| 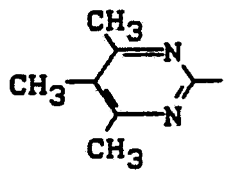 | 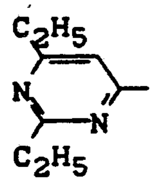 |
| 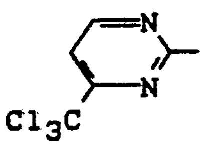 | |

Verwendet man beispielsweise 3,5-Dichlor-1,2,4-oxadiazol und 3-Nitro-2-mercaptopyridin als Ausgangsstoffe, so läßt sich der Reaktionsablauf des erfindungsgemäßen Verfahrens durch das folgende Formelschema darstellen:

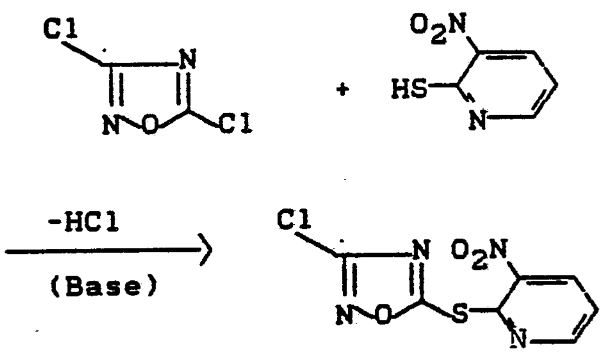


Das zur Durchführung des erfindungsgemäßen Verfahrens als Ausgangsstoff benötigte 3,5-Dichlor-1,2,4-oxadiazol der Formel (II) ist bekannt (vgl. DE-OS 34 45 205).

Die zur Durchführung des erfindungsgemäßen Verfahrens weiterhin als Ausgangsstoffe benötigten Thiole sind durch die Formel (III) allgemein definiert. In dieser Formel (III) steht R für diejenigen Reste, die bereits im Zusammenhang mit der Beschreibung der erfindungsgemäßen Stoffe der Formel (I) für diesen Substituenten genannt wurden.

Die Thiole der Formel (III) sind allgemein bekannte Verbindungen der organischen Chemie oder erhältlich in Analogie zu bekannten Verbindungen mit Hilfe allgemein bekannter Verfahren.

Als Verdünnungsmittel zur Durchführung des erfindungsgemäßen Verfahrens kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder -diethylester; Ketone, wie Aceton oder Butanon; Nitrile, wie Acetonitril oder Propionitril; Amide, wie Dimethylformamid, Dimethyl acetamid, N-Methylformanilid, N-Methylpyrrolidon oder Hexamethylphosphorsäuretriamid; Ester, wie Essigsäureethylester oder Sulfoxide, wie Dimethylsulfoxid.

Das erfindungsgemäße Verfahren kann gegebenenfalls in Gegenwart eines geeigneten Säurebindemittels durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin,

N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -20 °C und +140 °C, vorzugsweise bei Temperaturen zwischen +20 °C und +70 °C.

Zur Durchführung des erfindungsgemäßen Verfahrens setzt man pro Mol an 3,5-Dichlor-1,2,4-oxadiazol der Formel (II) im allgemeinen 0,7 bis 1,0 Mol, vorzugsweise äquimolare Mengen an Thiol der Formel (III) und 1,0 bis 1,5 Mol, vorzugsweise äquimolare Mengen an Base ein. Die Reaktionsdurchführung, Aufarbeitung und Isolierung der Reaktionsprodukte der Formel (I) erfolgt nach allgemein üblichen Verfahren.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel insbesondere als Fungizide und Bakterizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P, brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Pflanzenkrankheiten im Obst-und Getreideanbau, wie beispielsweise gegen den Erreger des Apfelschorfes (Venturia inaequalis) oder gegen den Erreger der Reisfleckenkrankheit (Pyricularia oryzae) einsetzen. Aufgrund ihrer systemischen Wirkung eignen sie sich insbesondere auch als Saatgutbeizmittel. Die breite fungizide Wirksamkeit der Verbindungen zeigt sich darüberhinaus auch bei in-Vitro-Tests.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver,

Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser also Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkei ten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

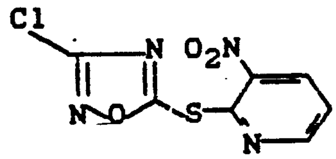

Zu 7,8 g (0,05 Mol) 3-Nitro-2-mercaptopyridin (vgl. Int. J. Pept. Prot. Res. 16 , 392 [1980]) und 6,9 g (0,05 Mol) Kaliumcarbonat in 100 ml wasserfreiem Acetonitril tropft man bei Raumtemperatur unter Rühren 7,0 g (0,05 Mol) 3,5-Dichlor-1,2,4-oxadiazol in 25 ml wasserfreiem Acetonitril. Nach beendeter Zugabe erhitzt man für 1 Stunde auf Rückflußtemperatur, gibt nach dem Abkühlen 300 ml Wasser zu, extrahiert dreimal mit je 200 ml Dichlormethan, wäscht die vereinigten organischen Phasen mit 200 ml Wasser, trocknet über Natriumsulfat und engt im Vakuum ein. Der Rückstand kristallisiert aus Diisopropylether.

Man erhält 10,2 g (79 % der Theorie) an 3-Chlor-5-(3-nitro-2-pyridylthio)-1,2,4-oxadiazol vom Schmelzpunkt 84 °C.

In entsprechender Weise and gemäß den allgemeinen Angaben zur Herstellung erhält man die folgenden 3-Chlor-1,2,4-oxadiazole der allgemeinen Formel (I):

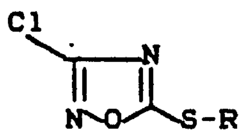 (I)

| Bsp.Nr. | R | Schmelzpunkt [°C] |
|---|---|---|
| 2 | 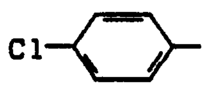 | Öl (Kp. 125° C-127° C/ 1,3 mbar) |
| 3 | 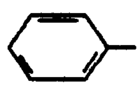 | Öl (Kp. 109° C-111° C/ 1,5 mbar) |
| 4 | 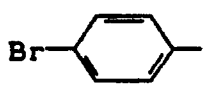 | 47-48 |
| 5 | 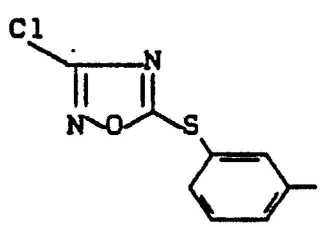 | 64-65 |
| 6 | 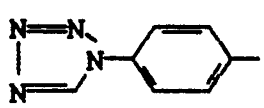 | 121-123 |

| Bsp.Nr. | R | Schmelzpunkt [°C] |
|---|---|---|
| 7 | 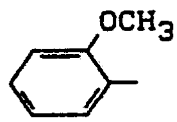 | 44-46 |
| 8 | 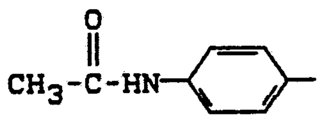 | 167-168 |
| 9 | 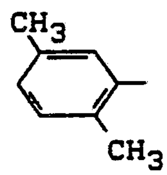 | 53-54 |
| 10 | 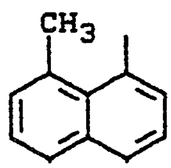 | 142-143 |
| 11 | 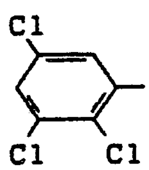 | Öl ($^1$H-NMR*): 7,4; 7,7) |
| 12 | 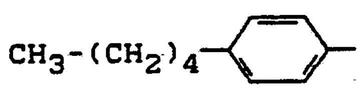 | Öl ($^1$H-NMR*): 1,7;2,7) |
| 13 | 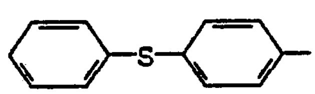 | 69-72 |
| 14 | 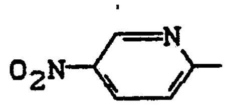 | 40-41 |

| Bsp.Nr. | R | Schmelzpunkt [°C] |
|---|---|---|
| 15 | 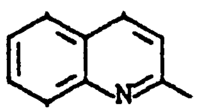 | 88-90 |
| 16 | 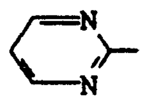 | 63-65 |
| 17 | 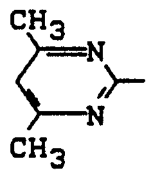 | 47-49 |
| 18 | 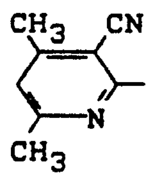 | 95-96 |
| 19 | 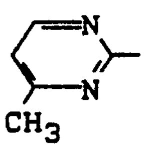 | Öl ($^1$H-NMR*): 8,5; 7,1; 2,5) |
| 20 | 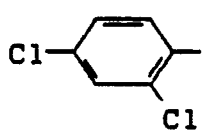 | Öl ($^1$H-NMR*): 7,3; 7,6; 7,7) |
| 21 | 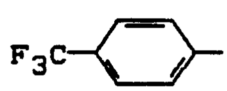 | Öl ($^1$H-NMR*): 7,75; 7,85) |
| 22 | 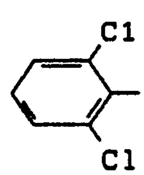 | 118-119 |

| Bsp.Nr. | R | Schmelzpunkt [°C] |
|---|---|---|
| 23 | 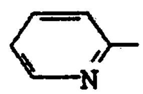 | Öl ($^1$H-NMR*): 1,4; 1,85; 8,6) |
| 24 | 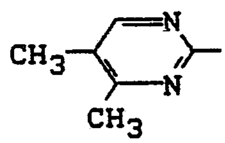 | Öl |
| 25 | 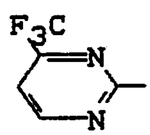 | Öl |
| 26 | 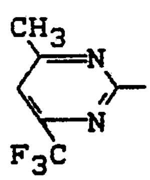 | Öl |
| 27 | 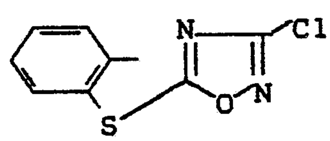 | Öl |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurde die nachstehend aufgeführte Verbindung als Vergleichsubstanz eingesetzt:

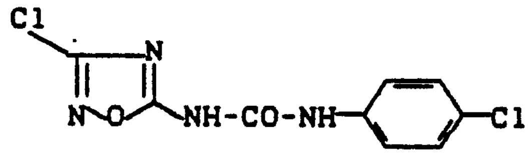

(A)

**N-(3-Chlor-1,2,4-oxadiazol-5-yl)-N'-(4-chlorphenyl)-harnstoff**

**(bekannt aus DE-OS 34 45 205).**

Beispiel A

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70% aufgestellt.

12 Tage nach der Inokulation erfolgt die Auswertung.

Eine deutliche Überlegenheit in der Wirksamkeit gegen-über dem Stand der Technik zeigen in diesem Test z.B. die Verbindungen gemäß den Herstellungsbeispielen: 5, 6, 8, 13, 15.

**Ansprüche**

1. 3-Chlor-1,2,4-oxadiazole der allgemeinen Formel

(I)

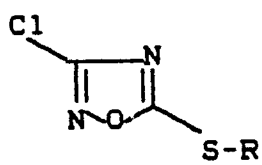

(I)

in welcher
R für gegebenenfalls substituiertes Aryl oder für gegebenenfalls substituiertes Heteroaryl steht.

2. 3-Chlor-1,2,4-oxadiazole der allgemeinen Formel (I) gemäß Anspruch 1, in welcher
R für gegebenenfalls einfach bis mehrfach, gleich oder verschieden substituiertes Aryl mit 6 bis 10 Kohlenstoffatomen oder für gegebenenfalls einfach bis mehrfach, gleich oder verschieden substituiertes und/oder benzannelliertes 5-bis 7-gliedriges Heteroaryl mit 1 bis 4 gegebenenfalls gleichen oder verschiedenen Heteroatomen steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Hydroxycarbonyl, Aminocarbonyl, geradkettiges oder verzweigtes Alkoxycarbonyl mit 1 bis 5 Kohlenstoffatomen, Aryloxy oder Arylthio mit jeweils 6 bis 10 Kohlenstoffatomen, jeweils geradkettiges oder verzweigtes Dialkylamino oder Alkylcarbonylamino mit jeweils 1 bis 4 Kohlenstoffatomen in den einzelnen Alkylteilen oder ein gegebenenfalls einfach bis mehrfach, gleich oder verschieden durch niederes Alkyl und/oder Halogen substituierter 5-Ring-oder 6-Ring-Heterocyclus mit 1 bis 4 gegebenenfalls gleichen oder verschiedenen Heteroatomen.

3. 3-Chlor-1,2,4-oxadiazole der Formel (I) gemäß Anspruch 1, in welcher
R für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-s-oder t-Butyl, n-oder i-Pentyl, Methoxy, Ethoxy, n-oder i-Propoxy, Methylthio, Ethylthio, n-oder i-Propylthio, Trifluormethyl, Trichlormethyl, Tribrommethyl, Trifluormethoxy, Trifluormethylthio, Hydroxycarbonyl, Aminocarbonyl, Methoxycarbonyl, Ethoxycarbonyl, Phenoxy, Phenylthio, Dimethylamino, Diethylamino, Methylcarbonylamino, Ethylcarbonylamino oder ein gegebenenfalls ein-oder zweifach, gleich oder verschieden durch Chlor, Brom, Methyl und/oder Ethyl substituierter 5-Ring-oder 6-Ring-Heterocyclus mit 1 bis 4 gegebenenfalls gleichen oder verschiedenen Heteroatomen; außerdem für jeweils gegebenenfalls ein-bis dreifach, gleich oder verschieden substituiertes α -Naphthyl oder β -Naphthyl steht, wobei als Substituenten infrage kommen: Fluor, Chlor, Brom, Nitro, Methyl, Ethyl, n-oder i-Propyl, n-, i-, s-oder t-Butyl, Methoxy, Ethoxy, Methylthio oder Trifluormethyl; außerdem für jeweils gegebenenfalls ein-bis dreifach, gleich oder verschieden substituiertes 2-Pyridyl, 4-Pyridyl, 2-Pyrimidyl, 4-Pyrimidyl, s-Triazinyl, 2-Chinolyl oder 1-Isochinolyl steht, wobei als Substituenten jeweils infrage kommen: Fluor, chlor, Brom, Cyano, Nitro, Methyl, Ethyl, n-oder i-Propyl, n-, i-, s-oder t-Butyl, n-oder i-Pentyl, Methoxy, Ethoxy, Methylthio, Trifluormethyl, Trichlormethyl, Tribrommethyl, Trifluormethoxy, Trifluormethylthio, Hydroxycarbonyl, Aminocarbonyl, Methoxycarbonyl, Ethoxycarbonyl, Dimethylamino, Methylcarbonylamino oder Ethylcarbonylamino.

4. 3-Chlor-1,2,4-oxadiazole der Formel (I) gemäß den Ansprüchen 1 bis 3, worin die Heteroatome in den Heteroarylen und Heterocyclen Stickstoff-, Sauerstoff-und Schwefelatome sind.

5. Verfahren zur Herstellung von 3-Chlor-1,2,4-oxadiazole der allgemeinen Formel (I),

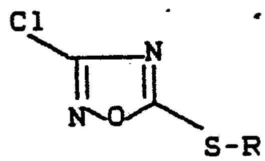

(I)

in welcher
R für gegebenenfalls substituiertes Aryl oder für gegebenenfalls substituiertes Heteroaryl steht,
dadurch gekennzeichnet, daß man 3,5-Dichlor-1,2,4-oxadiazol der Formel (II)

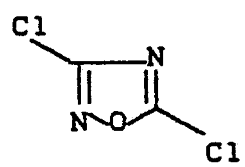

(II)

mit Thiolen der allgemeinen Formel (III),
HS-R (III)
in welcher
R die oben angegebene Bedeutung hat,
gegebenenfalls in Gegenwart eines Verdünnungsmittels und gegebenenfalls in Gegenwart eines Säurebindemittels umsetzt.

6. Schädlingsbekämpfungsmittel, gekennzeichnet durch einen Gehalt an mindestens einem 3-Chlor-1,2,4-oxadiazol der Formel (I) gemäß den Ansprüchen 1 und 5.

7. Verwendung von 3-Chlor-1,2,4-oxadiazolen der Formel (I) gemäß den Ansprüchen 1 und 5 zur Bekämpfung von Schädlingen.

8. Verfahren zur Bekämpfung von Schädlingen, dadurch gekennzeichnet, daß man 3-Chlor-1,2,4-oxadiazole der Formel (I) gemäß den Ansprüchen 1 und 5 auf Schädlinge oder ihren Lebensraum einwirken läßt.

9. Verfahren zur Herstellung von Schädlingsbekämpfungsmitteln, dadurch gekennzeichnet, daß man 3-Chlor-1,2,4-oxadiazole der Formel (I) gemäß den Ansprüchen 1 und 5 mit Streckmitteln und/oder oberflächenaktiven Mitteln vermischt.

10. Verwendung von 3-Chlor-1,2,4-oxadiazolen der Formel (I) gemäß Anspruch 7 zur Bekämpfung von Pilzen.

Europäisches Patentamt

# EUROPÄISCHER RECHERCHENBERICHT

Nummer der Anmeldung

EP 87 11 6766

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 185 983 (BAYER AG)<br>* Ansprüche 1, 5-8 * & DE - A - 3 445 205 (Kat. D)<br>--- | 1,6-9 | C 07 D 271/06<br>C 07 D 413/12<br>A 01 N 43/82 |
| A | DE-A-3 030 661 (BAYER)<br>* Ansprüche 1-7, 9, 10 *<br>--- | 1,6-9 | |
| A | DD-A- 222 770 (VEB CHEMIEKOMBINAT BITTERFELD)<br>----- | | |
| | | | **RECHERCHIERTE SACHGEBIETE (Int. Cl.4)** |
| | | | C 07 D 271/00<br>C 07 D 413/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 08-02-1988 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)